# EUROPÄISCHE PATENTSCHRIFT

(11) **EP 1 399 707 B1**
(45) Veröffentlichungstag und Bekanntmachung des Hinweises auf die Patenterteilung: **21.06.2006**
(21) Anmeldenummer: 02714116.7
(22) Anmeldetag: 25.01.2002
(51) Int. Cl.: G01B 7/28, A61B 17/16, G01S 5/16

(54) **VERFAHREN UND VORRICHTUNG ZUR BESTIMMUNG DER KONTUR EINER AUSNEHMUNG IN EINEM MATERIALSTÜCK**
METHOD AND DEVICE FOR DETERMINING THE CONTOUR OF A RECESS IN A PIECE OF MATERIAL
PROCEDE ET DISPOSITIF DE DETERMINATION DU CONTOUR D'UNE CAVITE PRATIQUEE DANS UNE PIECE

(30) Priorität: 07.02.2001 DE 10105822
(43) Veröffentlichungstag der Anmeldung: 24.03.2004
(73) Patentinhaber: Aesculap AG & Co. KG, 78532 Tuttlingen (DE)
(72) Erfinder: LEITNER, Francois Aesculap S.A., F-38100 Grenoble (FR); MOLLARD, Benoit Aesculap S.A., F-38100 Grenoble (FR); TÜMMLER, Hanns-Peter, 78532 Tuttlingen (DE)
(74) Vertreter: Hoeger, Stellrecht & Partner Patentanwälte
(86) Internationale Anmeldenummer: PCT/EP2002/000793
(87) Internationale Veröffentlichungsnummer: WO 2002/063236

(56) Entgegenhaltungen:
- WO-A-00/56215
- WO-A-99/15097
- WO-A-99/38449
- DE-A- 19 639 615
- DE-A- 19 951 502
- US-A- 6 006 126
- US-A- 6 106 464
- PATENT ABSTRACTS OF JAPAN vol. 2002, no. 02, 2. April 2002 (2002-04-02) & JP 2001 293006 A (OLYMPUS OPTICAL CO LTD), 23. Oktober 2001 (2001-10-23)

## Beschreibung

Die Erfindung betrifft ein Verfahren zur Bestimmung der Kontur einer Ausnehmung in einem Materialstück, die mit Hilfe eines Werkzeuges in das Material eingearbeitet wird, insbesondere in einem Knochenstück, bei dem man die Lage des Materialstückes im Raum durch ein Navigationssystem feststellt, die Lage des Werkzeuges im Raum durch ein Navigationssystem feststellt, aus den derart gewonnenen Positionsdaten die jeweilige Lage des Werkzeuges relativ zu dem Materialstück bestimmt und während der Bearbeitung des Materialstückes durch das Werkzeug die relativen Lagen des Werkzeuges relativ zum Materialstück speichert.

Die Erfindung betrifft weiterhin ein System umfassend ein Materialstück, ein Werkzeug zur Einarbeitung einer Ausnehmung in das Materialstück und eine Vorrichtung zur Bestimmung der Kontur der Ausnehmung in dem Materialstück, bei dem weiterhin das Materialstück und das Werkzeug jeweils fest mit einem Markierelement verbunden sind, und mit einem ortsfesten Navigationssystem zur Erfassung der Position der beiden Markierelemente und damit der Position des Materialstückes und des Werkzeuges im Raum sowie mit einer Recheneinheit mit Speicher zur Bestimmung der jeweiligen Lage des Werkzeuges relativ zu dem Materialstück aus den derart gewonnenen Positionsdaten und zur Speicherung der während der Bearbeitung des Materialstückes durch das Werkzeug eingenommenen relativen Lagen des Werkzeuges relativ zum Materialstück.

Es ist in vielfältiger Weise notwendig, in ein Materialstück eine Ausnehmung einer bestimmten Form und Größe einzubringen, beispielsweise in einem Knochenstück, in dem die Ausnehmung zur Aufnahme eines Implantates vorbereitet wird. Dabei ist es wichtig, die vorgegebene Kontur der Ausnehmung zu erreichen und diese vorgegebene Kontur nicht zu überschreiten.

Derartige Ausnehmungen werden in der Regel mit Spezialwerkzeugen hergestellt, beispielsweise mit rotierenden Formfräsern, mit Fingerfräsern, Bohrern oder ähnlichen Werkzeugen, aber auch mit handgeführten Werkzeugen, beispielsweise Messern, Sticheln, Feilen etc. In der Praxis ist es außerordentlich schwierig, in den ausgearbeiteten Ausnehmungen selbst zu erkennen, wie dort die Kontur verläuft, die von dem Werkzeug jeweils hergestellt worden ist. Dies wird einmal durch das abgetragene Material erschwert, zum anderen ist die Ausnehmung häufig schwer oder gar nicht zugänglich und daher nicht beobachtbar. In diesen Fällen ist man auf das Gefühl angewiesen, um die Größe und Form der Ausnehmung so gut wie möglich an die gewünschte Kontur anzupassen.

In der DE 199 51 502 A1 ist ein Navigationssystem beschrieben, das mit zwei Markierelementen arbeitet, nämlich einem Markierelement an einem Ultraschallsensor und einem zweiten Markierelement an einem Instrument. Damit ist es möglich, die relative Lage von Instrument und Ultraschallsensor zu bestimmen.

Ein anderes Navigationssystem, das mit zwei Markierelementen arbeitet, ist aus der WO 99/15097 bekannt. Dabei wird ein Markierelement an einem Körperteil befestigt, das andere an einem Instrument. Dadurch ist es möglich, die Lage des Instrumentes relativ zum Körperteil zu bestimmen.

Es ist Aufgabe der Erfindung, ein Verfahren anzugeben, mit dem es möglich ist, die tatsächliche Kontur der mit dem Werkzeug hergestellten Ausnehmung auch dann zu bestimmen, wenn die Ausnehmung nicht unmittelbar beobachtbar ist.

Diese Aufgabe wird bei einem Verfahren der eingangs beschriebenen Art erfindungsgemäß dadurch gelöst, daß man die Extremwerte dieser relativen Lagen bezüglich einer festen Bezugsposition des Materialstücks bestimmt und daß man aus diesen Extremwerten des Werkzeugs die ausgearbeitete Kontur am Materialstück bestimmt.

Bei diesem Verfahren wird also zunächst während des gesamten Bearbeitungsvorganges fortlaufend einmal die Lage des Materialstückes und zum anderen die Lage des Werkzeuges im Raum bestimmt. Dies läßt sich dadurch bewerkstelligen, daß ein sogenanntes Navigationssystem eingesetzt wird. Das sind an sich bekannte Systeme, die es ermöglichen, die Lage eines Gegenstandes im Raum genau zu bestimmen. Beispielsweise können sowohl am Materialstück als auch am Werkzeug Markierelemente befestigt werden, beispielsweise drei oder noch mehr Markierelemente, die als Sender oder Reflektor für eine elektromagnetische Strahlung dienen und die von einem ortsfesten Navigationssystem ausgesandte elektromagnetische Strahlung reflektieren und an das Navigationssystem zurücksenden bzw. eine solche Strahlung aussenden und an das Navigationssystem übermitteln. Aus den Laufzeitunterschieden der über mehrere Sender und Empfänger übermittelten Strahlung lassen sich die genauen Positionen der verschiedenen Markierelemente an dem Materialstück bzw. am Werkzeug bestimmen, und daraus kann die exakte Lage des Materialstückes und des Werkzeuges bestimmt werden.

Diese Positionsdaten ermöglichen es auch, die Relativposition des Werkzeuges und des Materialstückes zu bestimmen. Das Werkzeug hat dabei eine bestimmte Geometrie der bearbeitenden Bereiche, beispielsweise eines halbkugelförmigen Formfräsers, und diese Geometrie ist relativ zu dem am Werkzeug befestigten Markierelement immer gleich. Aus der Lagebestimmung des Markierelementes ist es also möglich, die Lage der bearbeitenden Fläche des Werkzeuges relativ zu dem zu bearbeitenden Materialstück zu bestimmen, und zwar fortlaufend während des gesamten Bearbeitungsvorganges.

Diese Positionsdaten, die die relative Lage von Werkzeug und Materialstück angeben, werden über den gesamten Bearbeitungsvorgang gespeichert. Aus diesen Daten können extreme Lagen des Werkzeuges relativ zum Materialstück bestimmt werden, und zwar relativ zu einer festen Position des Materialstückes. Mit anderen Worten kann beispielsweise ausgehend von einem festen Punkt des Materialstückes in einer bestimmten Richtung bestimmt werden, wann die zu bearbeitende Fläche des Werkzeuges einen maximalen Abstand von diesem Punkt gehabt hat. Dieser Abstand entspricht dann der tatsächlich in das Materialstück eingearbeiteten Kontur einer Ausnehmung, denn das Werkzeug bearbeitet das Material des Materialstückes immer bis zu dieser extremen Lage. Wenn der Abstand von dem festen Punkt geringer ist, berührt die bearbeitende Fläche des Werkzeuges das Material des Materialstückes nicht und entfernt auch kein Material.

Eine solche Bestimmung kann in unterschiedlichen Richtungen ausgehend von der festen Bezugsposition erfolgen, und auf diese Weise erhält man über die gesamte Oberfläche der eingearbeiteten Ausnehmung Informationen darüber, wo tatsächlich die Grenzfläche zwischen Ausnehmung und Material verläuft, wie weit also das Werkzeug jeweils Material aus dem Materialstück entfernt hat.

Diese Daten ermöglichen es dem Bearbeiter, genaue Informationen über die jeweilige Lage und Größe der Ausnehmung zu erhalten, auch wenn er die Ausnehmung selbst nicht beobachten kann.

Es ist dabei vorteilhaft, wenn man die Differenz der in dieser Weise bestimmten Kontur der ausgearbeiteten Ausnehmung relativ zu einer vorgegebenen Kontur an verschiedenen Stellen der Kontur bestimmt.

Bei diesem Verfahren geht man aus von einer gewünschten Kontur der Ausnehmung, deren Positionsdaten relativ zum Materialstück ebenfalls gespeichert werden können, diese Positionsdaten der gewünschten Kontur werden laufend mit den Positionsdaten der tatsächlich erarbeiten Kontur der Ausnehmung verglichen und zeigen, ob noch Material entfernt werden muß, um die gewünschte Kontur zu erreichen, ob die gewünschte Kontur bereits erreicht ist oder ob die gewünschte Kontur tatsächlich überschritten worden ist.

Bei einer besonders bevorzugten Ausführungsform ist vorgesehen, daß man die derart bestimmten Differenzen an verschiedenen Stellen der ausgearbeiteten Ausnehmung sichtbar darstellt, wobei es vorteilhaft ist, wenn man gleiche Differenzen an verschiedenen Stellen der ausgearbeiteten Ausnehmung in gleicher Weise darstellt, insbesondere in gleicher Farbe. Der Bearbeiter kann daher an einer solchen Darstellung unmittelbar ablesen, in welchen Bereichen der Ausnehmung noch eine Bearbeitung notwendig ist, um die gewünschte Kontur zu erreichen, in welchen Bereichen diese bereits erreicht ist, und in welchen Bereichen eventuell eine Überschreitung der gewünschten Kontur vorliegt.

Es ist auch vorteilhaft, wenn man der Abbildung der Kontur der Ausnehmung oder der festgestellten Differenz eine Abbildung des Materialstückes überlagert. Diese Abbildung kann dem Bearbeiter Auskunft über die Umgebung der Ausnehmung geben, so daß der Bearbeiter genau feststellen kann, in welchem Bereich des Materialstückes er arbeitet und ob er gegebenenfalls in Strukturen des Materialstückes eingreift, die nicht von der Ausnehmung erreicht werden sollen.

Insbesondere kann vorgesehen sein, daß man aus den bestimmten Extremwerten der relativen Lage ein Warnsignal erzeugt, wenn die Extremwerte bestimmte vorgegebene Maximalwerte überschreiten, wenn also die tatsächlich ausgearbeitete Ausnehmung in Bereiche des Materialstückes vorzudringen droht, in die ein Vordringen nicht gewünscht wird. Dies kann beispielsweise von Bedeutung sein, wenn in einen Knochen eine Ausnehmung zur Aufnahme eines Implantates eingearbeitet wird und wenn sichergestellt werden soll, daß diese Ausnehmung eine bestimmte Größe nicht überschreitet, etwa um ein Durchbrechen des Knochens an der Stelle der Ausnehmung zu verhindern.

Der Erfindung liegt auch die Aufgabe zugrunde, ein System der eingangs beschriebenen Art so zu verändern, daß ein Bearbeiter in die Lage versetzt wird, die Kontur der bearbeiteten Ausnehmung zu überwachen.

Diese Aufgabe wird bei einem System der eingangs beschriebenen Art erfindungsgemäß dadurch gelöst, daß die Recheneinheit derart programmiert ist, daß sie aus den gespeicherten relativen Lagen des Werkzeuges die Extremwerte dieser relativen Lagen bezüglich einer festen Bezugsposition des Materialstücks und aus diesen Extremwerten des Werkzeugs die ausgearbeitete Kontur am Materialstück bestimmt.

Weitere vorteilhafte Ausgestaltungen des Systems sind Gegenstand von Unteransprüchen.

Die nachfolgende Beschreibung bevorzugter Ausführungsformen der Erfindung dient im Zusammenhang mit der Zeichnung der näheren Erläuterung. Es zeigen:

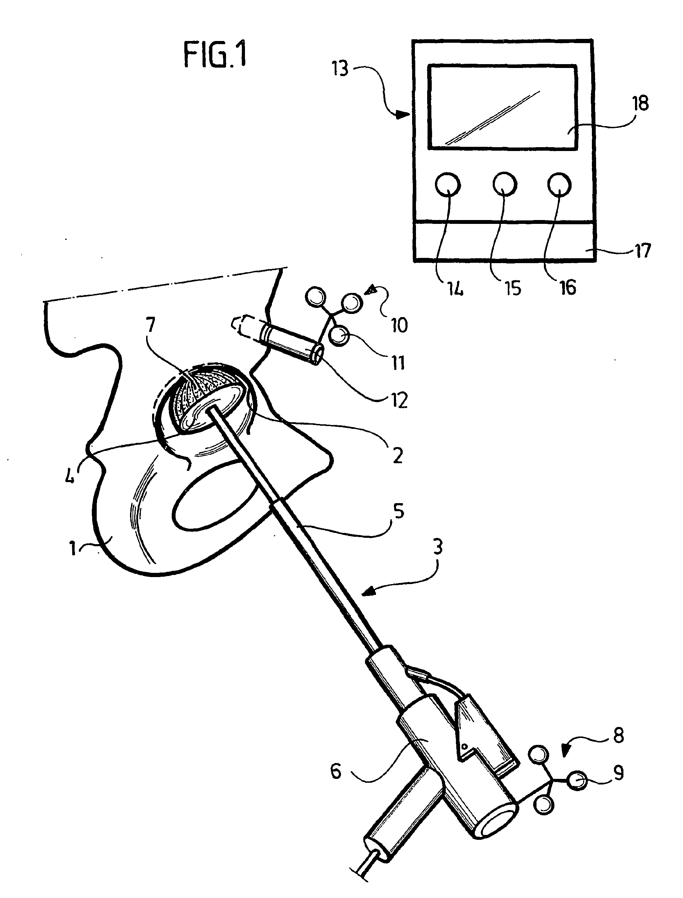

- Figur 1:: die Bearbeitung eines Materialstückes in Form eines Hüftknochens mit einem Fräswerkzeug unter Verwendung eines Navigationssystems;
- Figur 2:: eine Schnittansicht durch eine pfannenförmige Ausnehmung in einem Hüftknochen;
- Figur 3:: eine Ansicht ähnlich Figur 2 mit strichpunktiert dargestellten Fräswerkzeugen und
- Figur 4:: eine Bildschirmdarstellung einer pfannenförmigen Ausnehmung mit Darstellung unterschiedlich tief abgetragener Wandbereiche.

Die Erfindung wird nachstehend am Beispiel eines Hüftknochens 1 erörtert, in den eine etwa halbkugelige, pfannenförmige Ausnehmung 2 eingearbeitet werden soll, die der Aufnahme einer etwa halbkugeligen Pfanne eines Hüftgelenkimplantates dient, dieses Implantat ist in der Zeichnung nicht dargestellt. Die Erfindung ist besonders vorteilhaft anwendbar bei der Herstellung von Ausnehmungen in Knochenmaterial zu chirurgischen Zwecken, die Erfindung ist aber ohne weiteres auch verwendbar bei anderen Materialstücken, beispielsweise auch bei der Formgebung von Implantaten aus Knochenmaterial oder anderen Materialien selbst und daher nicht beschränkt auf die Bearbeitung von Materialstücken des menschlichen oder tierischen Körpers, wenn auch in diesem Bereich ein besonders vorteilhaftes Einsatzgebiet liegt.

Um in den Hüftknochen die pfannenförmige Ausnehmung 2 einarbeiten zu können, wird ein Werkzeug 3 verwendet, welches einen etwa halbkugelförmigen Formfräser 4 verwendet, der über eine starre Welle 5 mit einem Antriebsmotor verbunden ist, der in einem handgriffartigen Gehäuse 6 des Werkzeuges 3 aufgenommen ist. Durch den Antrieb wird die Welle 5 in Drehung versetzt und verdreht dabei den Formfräser 4 so, daß die halbkugelige Fräsfläche 7 des Formfräsers 4 in sich verdreht wird.

Das Werkzeug 3 trägt ein starr mit dem Gehäuse 6 verbundenes Markierelement 8 mit drei im Abstand zueinander angeordneten kugelförmigen Reflexionskörpern 9, ein gleichartiges Markierelement 10 mit ebenfalls drei im Abstand zueinander angeordneten Reflexionskörpern 11 ist mittels einer in den Hüftknochen 1 eingeschraubten Knochenschraube 12 starr mit diesem verbunden.

Ortsfest im Raum ist ein Navigationssystem 13 angeordnet, welches im dargestellten Ausführungsbeispiel drei Strahlungssender und -empfänger 14, 15, 16 aufweist, die beispielsweise eine Ultrarotstrahlung abstrahlen und diese nach Reflexion an den Reflexionskörpern 9 bzw. 11 wieder empfangen. Die Laufzeiten der Strahlung zwischen den Strahlungssendern und -empfängern 14, 15, 16 und den Reflexionskörpern 9 bzw. 11 der Markierelemente 8 und 10 wird in einer Rechen- und Speichereinheit 17 des Navigationssystems 13 bestimmt, und daraus können die genauen Positionen der Markierelemente 8 und 10 im Raum bestimmt werden, und diese Positionen sind eindeutig verbunden mit den Positionen des Hüftknochens 1 und des Werkzeuges 3 im Raum. Auf diese Weise kann das Navigationssystem 13 die exakte Positionierung sowohl des Hüftknochens 1 als auch des Werkzeuges 3 im Raum während des gesamten Bearbeitungsvorganges fortwährend feststellen.

Diesen jeweiligen Positionen des Hüftknochens 1 und des Werkzeuges 3 entsprechende Positionsdaten werden in der Rechen- und Speichereinheit 17 gespeichert, so daß nach Abschluß des Bearbeitungsvorganges die gesamte Bewegung des Hüftknochens 1 und des Werkzeuges 3 während des Bearbeitungsvorganges rekonstruierbar ist.

Außerdem werden in der Rechen- und Speichereinheit 17 die Positionsdaten des Hüftknochens 1 und des Werkzeuges 3 miteinander verglichen, und daraus wird berechnet, wo sich die Fräsfläche 7 des Werkzeuges 3 während der Bearbeitung relativ zum Hüftknochen 1 jeweils befindet. Da das Werkzeug 3 ein starrer Körper ist und da sich die Fräsfläche 7 bei der Drehung der starren Welle 5 in sich verdreht, bleibt die Einhüllende, die durch die Fräsfläche 7 gebildet wird, relativ zum übrigen Werkzeug 3 immer gleich und damit auch relativ zu dem entsprechenden Markierelement 8. Es ist daher ohne weiteres möglich, während des gesamten Bearbeitungsvorgangs die genaue Lage dieser einhüllenden Fräsfläche 7 zu bestimmen und sie in Beziehung zu bringen zu den Positionsdaten des Hüftknochens 1, welche über die Position des Markierelementes 10 erhalten werden.

Der Rechen- und Speichereinheit werden außerdem geometrische Daten des Hüftknochens 1 zur Verfügung gestellt, die beispielsweise durch eine Computertomographieaufnahme des Hüftknochens 1 erhalten werden und die die Form des Hüftknochens 1 beschreiben. Auf diese Weise kann die Rechen- und Speichereinheit 17 auch ausrechnen, ob die einhüllende Fräsfläche 7 in die Kontur des Hüftknochens 1 eindringt, wo dies exakt passiert und wie stark. Es wird also mit anderen Worten bestimmt, welcher Teil der Fräsfläche 7 die Außenkontur des Hüftknochens 1 durchdrungen hat. Dies ist ein Maß dafür, in welchem Bereich und in welchem Maße durch die Fräsfläche 7 Material aus dem Hüftknochen 1 entfernt worden ist, in diesem Bereich hat der Formfräser 4 eine Ausnehmung erzeugt.

Die Rechen- und Speichereinheit berechnet dieses Eindringen der Fräsfläche 7 in die Kontur des Hüftknochens 1 während des gesamten Bearbeitungsvorganges und speichert diese Daten. Gleichzeitig werden Extremwerte dieses Eindringens festgestellt, also die Positionsdaten, die dem weitesten Eindringen der Fräsfläche 7 in die Kontur des Hüftknochens 1 entsprechen. Dies kann beispielsweise dadurch erfolgen, daß ausgehend von einem fest angenommenen Punkt im Hüftknochen 1, insbesondere vom Mittelpunkt einer gewünschten halbkugeligen Ausnehmung 2 ausgehend, nach verschiedenen Richtungen die Abstände bestimmt werden, die die Fräsfläche 7 des Formfräsers 4 maximal erreicht hat. Erfaßt man diese Extremwerte in unterschiedlichen Richtungen über die gesamte Erstreckung der gewünschten Ausnehmung, so erhält man Positionsdaten, die die tatsächliche Grenzwand der erzeugten Ausnehmung 2 im Hüftknochen 1 angeben, also die tatsächliche Kontur der Ausnehmung 2.

Die so in der Rechen- und Speichereinheit 17 gewonnenen Positionsdaten der tatsächlichen Kontur der Ausnehmung 2 können auf einem Bildschirm 18 des Navigationssystems 13 sichtbar gemacht werden, so daß der Bearbeiter unmittelbar an dem Bildschirm 18 ablesen kann, wie weit der Formfräser 4 in den Hüftknochen 1 eingedrungen ist und welche Form die Ausnehmung 2 exakt hat.

Es sind hier verschiedene Darstellungsmöglichkeiten gegeben, beispielsweise könnten die jeweilig erreichten Tiefen der Ausnehmung 2 in einer Schnittansicht unmittelbar auf dem Bildschirm angezeigt werden, wobei die Schnittebenen variiert werden könnten. Es ist auch möglich, die Darstellung der Ausnehmung mit einer Abbildung des Hüftknochens zu überlagern, die aufgrund vorangehender Computertomographieaufnahmen gewonnen worden sind und in der Rechen- und Speichereinheit abgelegt sind. Auf diese Weise kann der Operateur direkt an einem Bild des Hüftknochens 1 erkennen, wo und wie die Ausnehmung 2 vom Formfräser 4 erzeugt wird.

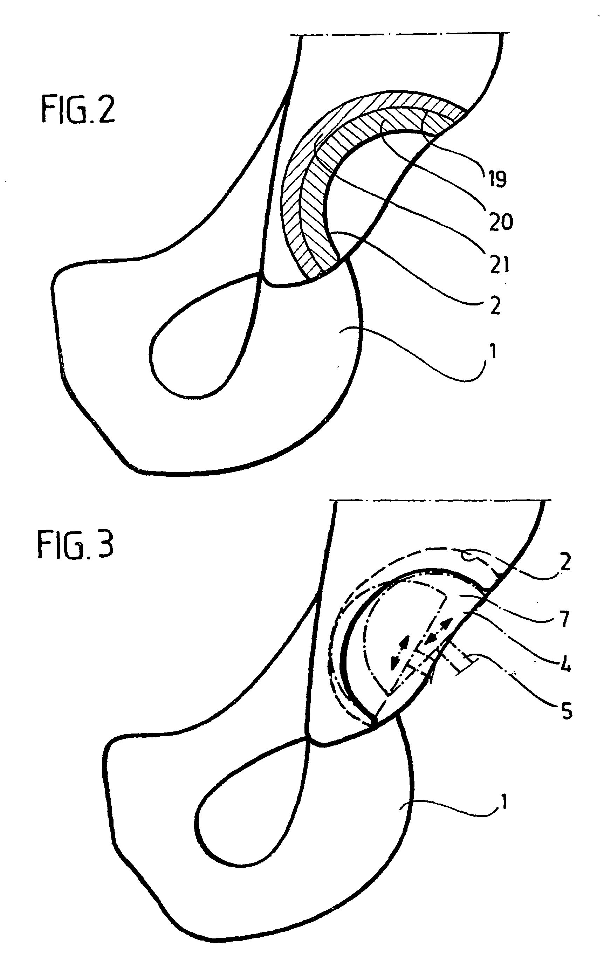

Bei einer bevorzugten Ausführungsform der Erfindung werden die Positionsdaten einer gewünschten Kontur der Ausnehmung 2 vorherbestimmt und in der Rechen- und Speichereinheit abgelegt. Beispielsweise kann diese gewünschte Kontur eine halbkugelförmige Kontur 19 sein, wie sie in der Schnittansicht der Figur 2 schematisch dargestellt ist. Bis zu dieser gewünschten Kontur 19 soll die Ausnehmung 2 von dem Formfräser 4 bearbeitet werden.

Dazu wird das Material des Hüftknochens 1 in konzentrische Schichten unterteilt, in Figur 2 sind dazu lediglich eine innenliegende Schicht 20 und eine außenliegende Schicht 21 angedeutet, die auf gegenüberliegenden Seiten der gewünschten Kontur 19 angeordnet sind.

Durch die Rechen- und Speichereinheit 17 wird über die gesamte Fläche der Ausnehmung 2 jeweils bestimmt, ob die tatsächlich von dem Formfräser 4 erzeugte Kontur innerhalb der innenliegenden Schicht 20, auf der gewünschten Kontur 19 oder in der außenliegenden Schicht 21 liegt, und in einer Ansicht 22 der kugelförmigen Ausnehmung 2, die zweidimensional oder auch dreidimensional gestaltet sein kann, werden die unterschiedlichen Bereiche unterschiedlich gekennzeichnet dargestellt, beispielsweise mit unterschiedlichen Farben. So werden Bereiche, in denen der Formfräser 4 die innenliegende Schicht 20 noch nicht erreicht hat, beispielsweise in der Ansicht 22 durch ein farbloses Gebiet 23 charakterisiert, Bereiche, in denen die Fräsfläche 7 bis in die innenliegende Schicht 20 vorgedrungen ist, durch ein grünes Gebiet 24 und Bereiche, in denen die Fräsfläche 7 die außenliegende Schicht 21 erreicht hat oder in diese eingedrungen ist, durch ein rotes Gebiet 25. Der Operateur kann daher an der Ansicht 22 sofort erkennen, wo im Bereich der dargestellten Ausnehmung 2 sich bei der Bearbeitung etwas ändert, dies ist ein Zeichen dafür, daß genau in diesem Bereich eine Bearbeitung und Vertiefung der Ausnehmung 2 erfolgt, außerdem kann er aber auch ablesen, ob sich die Fräsfläche 7 in dem gerade bearbeiteten Gebiet noch außerhalb der innenliegenden Schicht 20, bereits innerhalb der innenliegenden Schicht 20 oder gar bereits in der außenliegenden Schicht 21 befindet, dementsprechend kann das Werkzeug 3 unterschiedlich geführt werden, so daß die Ausnehmung 2 so bearbeitet werden kann, daß die tatsächliche Ausnehmung 2 nach Beendigung des Bearbeitungsvorganges optimal an die gewünschte Kontur 19 angepaßt ist.

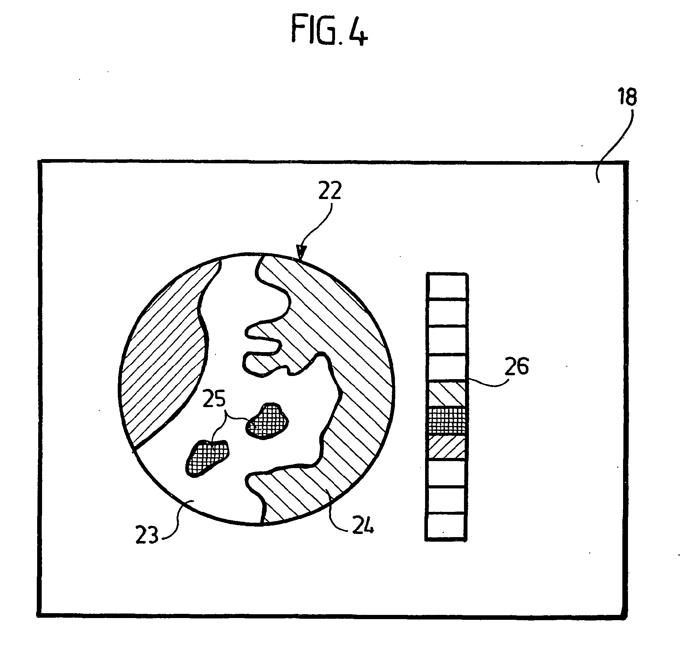

Bei der Darstellung der Figur 4 ist neben der Ansicht 22 auch noch die jeweilige Dicke der Schichten 20 und 21 in einer neben der Ansicht 22 angeordneten Skala 26 angegeben, tatsächlich kann natürlich die Zahl der entsprechenden Schichten auch wesentlich höher sein, so daß ein sehr feinfühliges Abarbeiten des Materials möglich wird.

Durch die in der Rechen- und Speichereinheit 17 gespeicherten Positionsdaten kann gleichzeitig ein Protokoll des Bearbeitungsvorganges erstellt werden, so daß nach Abschluß der Bearbeitung des Hüftknochens 1 genau festgestellt werden kann, wie das Werkzeug 3 während des Bearbeitungsvorganges geführt worden ist und ob die Ausnehmung. 2 insgesamt der gewünschten Kontur 19 entspricht.

Die jeweils festgestellten Positionsdaten des Formfräsers 4 können auch dazu verwendet werden, ein unbeabsichtigte, zu tiefes Eindringen des Formfräsers 4 in den Hüftknochen 1 zu verhindern, es können hier Grenzwerte für das Eindringen festgelegt werden, falls diese in irgendeinem Gebiet der Ausnehmung 2 erreicht werden, kann ein Warnsignal oder ein Abschaltsignal erzeugt werden.

Durch das beschriebene Verfahren ist es möglich, auch unwillkürliche und in der Praxis nicht zu vermeidende Abweichbewegungen des Formfräsers 4 bei der Bearbeitung zu kontrollieren, zu protokollieren und gegebenenfalls auszugleichen, da der Benutzer auf dem Bildschirm 18 den Fortgang des Bearbeitungsvorganges genau verfolgen und das Werkzeug 3 abhängig vom Fortgang des Bearbeitungsvorganges führen kann. Dies ist möglich, obwohl ein direkter Sichtzugang zur Bearbeitungsstelle fehlt, es ist auch nicht notwendig, eine solche Sicht über Röntgenstrahlbeobachtung herzustellen.

## Patentansprüche

1. Verfahren zur Bestimmung der Kontur einer Ausnehmung in einem Materialstück, die mit Hilfe eines Werkzeuges in das Material eingearbeitet wird, insbesondere in einem Knochenstück, bei dem man die Lage des Materialstückes im Raum durch ein Navigationssystem feststellt, die Lage des Werkzeuges im Raum durch ein Navigationssystem feststellt, aus den derart gewonnenen Positionsdaten die jeweilige Lage des Werkzeuges relativ zu dem Materialstück bestimmt und während der Bearbeitung des Materialstückes durch das Werkzeug die relativen Lagen des Werkzeuges relativ zum Materialstück speichert, **dadurch gekennzeichnet, daß** man die Extremwerte dieser relativen Lagen bezüglich einer festen Bezugsposition des Materialstücks bestimmt und daß man aus diesen Extremwerten des Werkzeugs die ausgearbeitete Kontur am Materialstück bestimmt.

2. Verfahren nach Anspruch 1, **dadurch gekennzeichnet, daß** man die ausgearbeitete Kontur sichtbar darstellt.

3. Verfahren nach einem der voranstehenden Ansprüche, **dadurch gekennzeichnet, daß** man die Differenz der in dieser Weise bestimmten Kontur der ausgearbeiteten Ausnehmung relativ zu einer vorgegebenen Kontur an verschiedenen Stellen der Kontur bestimmt.

4. Verfahren nach Anspruch 3, **dadurch gekennzeichnet, daß** man die derart bestimmten Differenzen an verschiedenen Stellen der ausgearbeiteten Ausnehmung sichtbar darstellt.

5. Verfahren nach Anspruch 4, **dadurch gekennzeichnet, daß** man gleiche Differenzen an verschiedenen Stellen der ausgearbeiteten Ausnehmung in gleicher Weise darstellt.

6. Verfahren nach Anspruch 5, **dadurch gekennzeichnet, daß** man gleiche Differenzen in gleicher Farbe anzeigt.

7. Verfahren nach einem der Ansprüche 2, 5 oder 6, **dadurch gekennzeichnet, daß** man der Abbildung der Kontur der Ausnehmung oder der festgestellten Differenz eine Abbildung des Materialstückes überlagert.

8. Verfahren nach einem der voranstehenden Ansprüche, **dadurch gekennzeichnet, daß** man aus den bestimmten Extremwerten der relativen Lage ein Warnsignal erzeugt, wenn diese Extremwerte bestimmte vorgegebene Maximalwerte überschreiten.

9. System umfassend ein Materialstück (1), ein Werkzeug (3) zur Einarbeitung einer Ausnehmung in das Materialstück (1) und eine Vorrichtung zur Bestimmung der Kontur der Ausnehmung in dem Materialstück (1), bei dem weiterhin das Materialstück (1) und das Werkzeug (3) jeweils fest mit einem Markierelement (10, 8) verbunden sind, und mit einem ortsfesten Navigationssystem (13) zur Erfassung der Position der beiden Markierelemente (10; 8) und damit der Position des Materialstückes (1) und des Werkzeuges (3) im Raum sowie mit einer Recheneinheit mit Speicher (17) zur Bestimmung der jeweiligen Lage des Werkzeuges (3) relativ zu dem Materialstück (1) aus den derart gewonnenen Positionsdaten und zur Speicherung der während der Bearbeitung des Materialstückes (1) durch das Werkzeug (3) eingenommenen relativen Lagen des Werkzeuges (3) relativ zum Materialstück (1), **dadurch gekennzeichnet, daß** die Recheneinheit derart programmiert ist, daß sie aus den gespeicherten relativen Lagen des Werkzeuges (3) die Extremwerte dieser relativen Lagen bezüglich einer festen Bezugsposition des Materialstücks (1) und aus diesen Extremwerten des Werkzeugs (3) die ausgearbeitete Kontur am Materialstück (1) bestimmt.

10. System nach Anspruch 9, **dadurch gekennzeichnet, daß** ein Sichtgerät (18) vorgesehen ist, welches die ausgearbeitete Kontur sichtbar darstellt.

11. System nach einem der Ansprüche 9 oder 10, **dadurch gekennzeichnet, daß** die Recheneinheit mit Speicher (17) die Differenz der in dieser Weise bestimmten Kontur der ausgearbeiteten Ausnehmung (2) relativ zu einer vorgegebenen Kontur (19) an verschiedenen Stellen der Kontur bestimmt.

12. System nach Anspruch 11, **dadurch gekennzeichnet, daß** die Recheneinheit mit Speicher (17) die derart bestimmten Differenzen an verschiedenen Stellen der ausgearbeiteten Ausnehmung (2) sichtbar darstellt.

13. System nach Anspruch 12, **dadurch gekennzeichnet, daß** die Recheneinheit mit Speicher (17) gleiche Differenzen an verschiedenen Stellen der ausgearbeiteten Ausnehmung (2) in gleicher Weise darstellt.

14. System nach Anspruch 13, **dadurch gekennzeichnet, daß** die Recheneinheit mit Speicher (17) gleiche Differenzen in gleicher Farbe anzeigt.

15. System nach einem der Ansprüche 9 bis 14, **dadurch gekennzeichnet, daß** die Recheneinheit mit Speicher (17) der Abbildung der Kontur der Ausnehmung (2) oder der festgestellten Differenz eine Abbildung des Materialstückes (1) überlagert.

16. System nach einem der Ansprüche 9 bis 15, **dadurch gekennzeichnet, daß** die Recheneinheit mit Speicher (17) aus den bestimmten Extremwerten der relativen Lage ein Warnsignal erzeugt, wenn diese Extremwerte bestimmte vorgegebene Maximalwerte überschreiten.

## Claims

1. Method for determining the contour of a recess in a piece of material, which is worked into the material by means of a tool, in particular in a piece of bone, in which the position of the piece of material in space is established by a navigation system, the position of the tool in space is established by a navigation system, the current position of the tool in relation to the piece of material is determined from the position data obtained in this manner, and during the machining of the piece of material with the tool, the relative positions of the tool in relation to the piece of material are stored **characterised in that** the extreme values of these relative positions with reference to a fixed reference position of the piece of material are determined and **in that** the prepared contour on the piece of material is determined from these extreme values of the tool.

2. Method according to Claim 1, **characterised in that** the prepared contour is visually displayed.

3. Method according to one of the preceding claims, **characterised in that** the difference of the contour of the prepared recess determined in this manner from a predetermined contour is determined at different locations of the contour.

4. Method according to Claim 3, **characterised in that** the differences determined in this manner at different locations of the prepared recess are visually displayed.

5. Method according to Claim 4, **characterised in that** the same differences at different locations of the prepared recess are displayed in the same manner.

6. Method according to Claim 5, **characterised in that** the same differences are displayed in the same colour.

7. Method according to one of Claims 2, 5 or 6, **characterised in that** an image of the piece of material is superposed on the image of the contour of the recess or of the detected difference.

8. Method according to one of the preceding claims, **characterised in that** from the determined extreme values of the relative position, a warning signal is generated when these extreme values exceed specific given maximum values.

9. System comprising a piece of material (1), a tool (3) for working a recess into the piece of material (1) and a device for determining the contour of the recess in the piece of material (1), in which, furthermore, the piece of material (1) and the tool (3) are respectively firmly connected to a marking element (10, 8), and with a stationary navigation system (13) for establishing the position of the two marking elements (10; 8) and thus the position of the piece of material (1) and the tool (3) in space, and with an arithmetic unit with memory (17) for determining the current position of the tool (3) in relation to the piece of material (1) from the position data obtained in this manner and for storing the relative positions of the tool (3) in relation to the piece of material (1) during machining of the piece of material (1) by the tool (3), **characterised in that** the arithmetic unit is programmed such that, from the stored relative positions of the tool (3) it determines the extreme values of these relative positions with reference to a fixed reference position of the piece of material (1) and determines the prepared contour on the piece of material (1) from these extreme values of the tool (3).

10. System according to Claim 9, **characterised in that** a display device (18) is provided which visually displays the prepared contour.

11. System according to one of Claims 9 or 10, **characterised in that** the arithmetic unit with memory (17) determines the difference of the contour of the prepared recess (2) determined in this manner from a predetermined contour (19) at different locations of the contour.

12. System according to Claim 11, **characterised in that** the arithmetic unit with memory (17) visually displays the differences determined in this manner at different locations of the prepared recess (2).

13. System according to Claim 12, **characterised in that** the arithmetic unit with memory (17) displays the same differences at different locations of the prepared recess (2) in the same manner.

14. System according to Claim 13, **characterised in that** the arithmetic unit with memory (17) displays the same differences in the same colour.

15. System according to one of Claims 9 to 14, **characterised in that** the arithmetic unit with memory (17) superposes an image of the piece of material (1) on the image of the contour of the recess (2) or of the established difference.

16. System according to one of Claims 9 to 15, **characterised in that** from the determined extreme values of the relative position, the arithmetic unit with memory (17) generates a warning signal when these extreme values exceed specific predetermined maximum values.

## Revendications

1. Procédé de détermination du contour d'une cavité pratiquée dans un fragment de matière, cavité qui est réalisée à l'aide d'un outil dans la matière, en particulier dans un fragment d'os dans lequel on détermine la position du fragment de matière dans l'espace par un système de navigation, on détermine la position de l'outil dans l'espace par un système de navigation, on détermine la position respective de l'outil par rapport au fragment de matière à partir des données sur la position ainsi obtenues et on enregistre, pendant l'usinage du fragment de matière par l'outil, les positions relatives de l'outil par rapport au fragment de matière, **caractérisé en ce que** l'on détermine les valeurs extrêmes de ces positions relatives par rapport à une position de référence fixe du fragment de matière et **en ce que** l'on détermine, à partir de ces valeurs extrêmes de l'outil, le contour façonné sur le fragment de matière.

2. Procédé selon la revendication 1, **caractérisé en ce que** l'on représente de manière visible le contour façonné.

3. Procédé selon l'une quelconque des revendications précédentes, **caractérisé en ce que** l'on détermine la différence du contour déterminé de cette manière de la cavité réalisée par rapport à un contour prédéterminé à différents endroits du contour.

4. Procédé selon la revendication 3, **caractérisé en ce que** l'on représente de manière visible les différences ainsi déterminées à différents endroits de la cavité façonnée.

5. Procédé selon la revendication 4, **caractérisé en ce que** l'on représente de la même manière les mêmes différences à différents endroits de la cavité façonnée.

6. Procédé selon la revendication 5, **caractérisé en ce que** l'on affiche dans une même couleur les mêmes différences.

7. Procédé selon l'une quelconque des revendications 2, 5 ou 6 **caractérisé en ce que** l' on associe à l'illustration du contour de la cavité ou à la différence constatée, une illustration du fragment de matière.

8. Procédé selon l'une quelconque des revendications précédentes, **caractérisé en ce que** l'on produit un signal d'avertissement à partir des valeurs extrêmes déterminées de la position relative lorsque ces valeurs extrêmes dépassent certaines valeurs maximales prédéfinies.

9. Système comportant un fragment de matière (1), un outil (3) pour la réalisation d'une cavité dans le fragment de matière (1) et un dispositif de détermination du contour de la cavité dans le fragment de matière (1), dans lequel, en outre, le fragment de matière (1) et l'outil (3) sont chacun solidement raccordés à un élément de marquage (10, 8), et un système de navigation stationnaire (13) pour la détection de la position des deux éléments de marquage (10 ; 8) et ainsi de la position du fragment de matière (1) et de l'outil (3) dans l'espace ainsi qu'à une unité de calcul avec mémoire (17) pour la détermination de la position respective de l'outil (3) par rapport au fragment de matière (1) à partir des données de position ainsi obtenues et pour l'enregistrement des positions relatives de l'outil (3), adoptées pendant l'usinage du fragment de matière (1) par l'outil (3), par rapport au fragment de matière (1), **caractérisé en ce que** l'unité de calcul est programmée de telle sorte qu'elle détermine, à partir des positions relatives enregistrées de l'outil (3), les valeurs extrêmes de ces positions relatives par rapport à une position de référence fixe du fragment de matière (1) et, à partir de ces valeurs extrêmes de l'outil (3), le contour façonné sur le fragment de matière (1).

10. Système selon la revendication 9, **caractérisé en ce qu'**un appareil de visualisation (18) est prévu, lequel représente de manière visible le contour façonné.

11. Système selon l'une quelconque des revendications 9 ou 10, **caractérisé en ce que** l'unité de calcul avec mémoire (17) détermine la différence du contour, déterminé de cette manière, de la cavité (2) façonnée par rapport à un contour prédéfini (19), à différents endroits du contour.

12. Système selon la revendication 11, **caractérisé en ce que** l'unité de calcul avec mémoire (17) représente de manière visible les différences ainsi déterminées, à différents endroits de la cavité (2) façonnée.

13. Système selon la revendication 12, **caractérisé en ce que** l'unité de calcul avec mémoire (17) représente de la même manière les mêmes différences, à différents endroits de la cavité (2) façonnée.

14. Système selon la revendication 13, **caractérisé en ce que** l'unité de calcul avec mémoire (17) affiche les mêmes différences dans la même couleur.

15. Système selon l'une quelconque des revendications 9 à 14, **caractérisé en ce que** l'unité de calcul avec mémoire (17) associe à l'illustration du contour de la cavité (2) ou de la différence constatée, une illustration du fragment de matière (1).

16. Système selon l'une quelconque des revendications 9 à 15, **caractérisé en ce que** l'unité de calcul avec mémoire (17) produit un signal d'avertissement à partir des valeurs extrêmes déterminées de la position relative lorsque ces valeurs extrêmes dépassent certaines valeurs maximales prédéfinies.
